# EUROPÄISCHE PATENTSCHRIFT

(11) **EP 1 181 381 B1**
(45) Veröffentlichungstag und Bekanntmachung des Hinweises auf die Patenterteilung: **20.10.2004**
(21) Anmeldenummer: 00925261.0
(22) Anmeldetag: 04.05.2000
(51) Int. Cl.: C12N 15/86, A61K 39/00

(54) **ORGAN-, GEWEBS- UND ZELLSPEZIFISCHES IMMUNTHERAPEUTIKUM FÜR CHRONISCHE VIRALE INFEKTIONEN, SOWIE ENTZÜNDLICHE, DEGENERATIVE UND PROLIFERATIVE ERKRANKUNGEN INSBESONDERE DER LEBER SOWIE KREBS AUF DER BASIS VON REKOMBINANTEM PARAPOXVIRUS**
ORGAN, TISSUE AND CELL-SPECIFIC IMMUNO-THERAPEUTIC FOR CHRONIC VIRAL INFECTIONS AND INFLAMMATORY, DEGENERATIVE AND PROLIFERATIVE DISEASES, IN PARTICULAR OF THE LIVER, AND FOR CANCER, BASED ON A RECOMBINANT PARAPOX VIRUS
AGENT IMMUNO-THERAPEUTIQUE SPECIFIQUE DES ORGANES, TISSUS ET CELLULES DES INFECTIONS VIRALES CHRONIQUES, DES AFFECTIONS PROLIFERATIVES, DEGENERATIVES ET INFLAMMATOIRES, EN PARTICULIER DU FOIE, ET DU CANCER, A BASE DE PARAPOXVIRUS RECOMBINE

(30) Priorität: 14.05.1999 DE 19922407
(43) Veröffentlichungstag der Anmeldung: 27.02.2002
(73) Patentinhaber: Bayer HealthCare AG, 51368 Leverkusen (DE)
(72) Erfinder: WEBER, Olaf, D-42489 Wülfrath (DE); SIEGLING, Angela, D-42115 Wuppertal (DE); SCHLAPP, Tobias, D-51061 Köln (DE)
(86) Internationale Anmeldenummer: PCT/EP2000/004011
(87) Internationale Veröffentlichungsnummer: WO 2000/069455

(56) Entgegenhaltungen:
- WO-A-97/37031
- DE-A- 19 639 601
- DE-C- 4 405 841
- J. HARRIS ET AL.: "Strategies for targeted gene therapy" TRENDS IN GENETICS, Bd. 12, Nr. 10, 1996, Seiten 400-405, XP000961250
- H.J. RHIZA ET AL.: "parapoxviruses: potential alternative vectors for directing the immune response in permissive and non-permissive hosts" J. BIOTECHNOL., Bd. 73, 1999, Seiten 235-242, XP000961259 in der Anmeldung erwähnt
- M. BÜTTNER AT AL.: "Interferon induction in peripheral blood mononuclear leukocytes of man and farm animals by poxvirus vector candidates and some poxvirus constructs" VET. IMMUNOL. IMMUNOPATHOL., Bd. 46, 1995, Seiten 237-250, XP000961253 in der Anmeldung erwähnt

## Beschreibung

Die vorliegende Erfindung betrifft die Herstellung und den Einsatz von rekombinantem Parapoxvirus zur organ-, gewebs-und/oder zellspezifisch zielgerichteten Immuntherapie von viralen Infektionen, sowie entzündlichen, degenerativen, proliferativen Erkrankungen, insbesondere der Leber; und Krebs. Sie betrifft weiter die Verwendung von rekombinantem Parapoxvirus mit Targetingeigenschaften zur Herstellung von Arzneimitteln.

In den Anwendungsbereich der oben genannten Parapoxviren fallen auch Erkrankungen der Haut und ihrer Anhangsgebilde, der inneren Organe, des Zentralnervensystems und seiner Anhangsgebilde einschließlich des Auges, sowie Krebs, bei Mensch und Tier.

Es ist bekannt, dass latente und chronisch persistente virale Infektionen durch eine Immunsuppression aktiviert bzw. reaktiviert werden können, oder umgekehrt, dass das Immunsystem die akute Erkrankung, die durch ein Virus, das latent ist, hervorgerufen werden kann, unterdrückt (z.B. rekurriert eine latente Herpesvirus-Infektion bei Immunsuppression: Lippenbläschen bei Stress oder Kortisongabe). Es ist weiter bekannt, dass chronisch persistente und latente virale Infektionen schwer oder garnicht mit herkömmlichen antiviralen Substanzen auf niedermolekularer Basis therapierbar sind.

Ein Grund dafür kann die fehlende virale enzymatische Aktivität bei solchen Infektionen sein (beispielsweise das Fehlen einer viralen Polymerase-Aktivität, die einen nukleosidischen Inhibitor erst in die virale Nukleinsäure einbauen muss, damit dieser z.B. zum Kettenabbruch in der viralen DNA führen kann; beispielsweise das Fehlen einer viralen Thymidinkinase-Aktivität, die z.B. eine antivirale Verbindung erst phosphorylieren muss, damit diese aktiv werden kann) oder aber die fehlende Erkennung infizierter Zellen oder viraler Antigene durch das Immunsystem des Wirtes.

Bekannt ist ebenfalls, dass bei chronisch persistierenden viralen Infektionen eine Superinfektion mit einem anderen Virus zu antiviralen, gegen das chronisch persistierende Virus gerichteten Effekten führen kann. 1) Die Abhängigkeit dieses Effektes von Interferonen (v.a. IFN-γ) und TNF-α, die von T-Zellen, natürlichen Killerzellen und Makrophagen sezemiert werden, konnte von den Autoren gezeigt werden.

Die Ergebnisse dieser Autoren bestätigte eine andere frühere Studie, in der gezeigt wurde, dass Class-I-restringierte cytotoxische T-Zellen die hepatozelluläre HBV-Genexpression in HBV- transgenen Mäusen hemmen konnten, dass dieser Prozess ohne Zerstörung der Leberzellen ablief und dass der Prozess durch TNF-α und IFN-γ-hervorgerufen wurde ²⁾.

In der tiermedizinischen Praxis wird seit längerer Zeit ein Produkt zur Induktion "paraspezifischer Immunität", ein sogenannter Paramunitätsinducer therapeutisch, meta- und prophylaktisch eingesetzt. Diese Paramunitätsinducer bestehen z.B. aus chemisch inaktiviertem Parapoxvirus ovis. Ein auf der Basis dieses Virus (Parapoxvirus ovis, Strain D 1701) hergestelltes Produkt ist BAYPAMUN® (DE 3504940).

Das inaktivierte Virus induziert im Tier einen unspezifischen Schutz gegenüber Infektionen mit den verschiedensten Erregern. Man nimmt an, dass dieser Schutz über verschiedene Mechanismen des organismus-eigenen Abwehrsystems vermittelt wird.

Dazu zählen: Induktion von Interferon, Aktivierung der natürlichen Killerzellen, Induktion der "Kolonien-Stimulierenden Aktivität" (CSA), sowie Stimulierung der Lymphozytenproliferation. Frühere Untersuchungen zum Wirkmechanismus zeigten die Stimulation von Interleukin 2 und Interferon-γ³⁾.

Bekannt ist ebenfalls, dass Parapoxviren als Vektoren mit Genen von anderen Erregern versehen werden können, um entsprechende Proteine exprimieren zu können und so einen prophylaktischen Immunschutz (Vaccinierung) gegen den Spendererreger zu erzeugen. ⁴⁾

Es ist weiter bekannt, dass rekombinante sogenannte "pseudotyped" Viren ursprünglich nicht infizierbare Ziel-Zellen, -Gewebe, -Organe bzw. -Wirte infizieren können ⁵⁾.

Basierend auf solchen Erkenntnissen wurde bereits der zielgerichtete Einsatz von gentherapeutischen Vektoren diskutiert ⁶⁾.

In der Pharmakologie bedient man sich natürlicher und synthetischer Moleküle, wie beispielsweise des Asialofetuin bzw. poly-L-Lysin, um bestimmte Organe- - im Falle der hier genannten Beispiele die Leber- aufgrund der Wechselwirkung mit organspezifischen Rezeptoren - im Falle der hier genannten Beispiele des Asialoglycoproteinrezeptors der Leber - mit diesen Molekülen selektiv für eine Therapie zugänglich zu machen ⁷⁾.

Vor diesem Hintergrund stellt sich daher die Aufgabe, die therapeutische Nutzbarkeit der ausgezeichneten immunogenen Wirkung von Parapoxvirus ovis dahingehend weiter zu verbessern, dass die oben beschriebene generalisierte paraspezifische Immunogenität des Parapoxvirus gezielt auf das erkrankte Organ-(system) und den Krankheits-Erreger gelenkt werden kann.

Eine solche Fokussierung ließe einen nebenwirkungsärmeren und am Wirkort stärkeren und nachhaltigeren therapeutischen Effekt erwarten.

Aufgabe der Erfindung war es deshalb, die immunologische Wirkung des Parapoxvirus zielgerichtet zu erzeugen. Die Aufgabe wird gelöst durch Koppeln oder das Einführen geeigneter Fremd-Peptide oder Proteine an bzw. in das Virus mit der Fähigkeit zur Interaktion mit Organ-, Gewebe- und/oder zellspezifischen Rezeptormolekülen.

So erreichten wir eine starke Fokussierung der Immunreaktion. Damit wird es erstmals möglich, mit Hilfe von Parapoxvirus ovis die komplexe Kapazität des Immunsystems am Ort des Bedarfs zu konzentrieren.

Die sich daraus ergebenden Vorteile bestehen in der Gewebe-, Organ-, bzw. Zellspezifität bei gleichzeitiger Verstärkung der immunologischen Wirkung am Ort des Bedarfs und in der Verringerung von Nebenwirkungen.

Da man mit den bisher bekannten Methoden/Produkten bei systemischer Applikation einerseits unerwünschte Nebenwirkungen allgemeiner Art in Kauf nehmen muss und/oder andererseits nur eine ungenügende Konzentration des Wirkstoffs am Wirkort erreicht, kann man mit der hier dargestellten neuen Qualität von Parapoxvirus ovis gezielter und effektiver therapieren.

Für die Herstellung von rekombinantem Parapoxvirus ovis zur zielgerichteten organ-, gewebs- und/oder zell-spezifischen Immuntherapie kann man bekannte virale Proteine/Peptide verwenden, die sowohl unmodifiziert als auch modifiziert, verlängert, oder verkürzt sein können. Als in diesem Zusammenhang besonders geeignet hat sich dabei z.B. das große Hüllprotein des humanen Hepatitis B Virus (HBV) Zum Erreichen der Leber erwiesen.

Weiter können nichtvirale Proteine/Peptide, insbesondere das Asialoglycoprotein, zur zielgerichteten Therapie der Leber verwendet werden.

Möglich ist auch die Verwendung neuer synthetischer Proteine/Peptide deren Sequenzen mittels dem Fachmann geläufiger Techniken zum Beispiel aus Phagen-Bibliotheken identifiziert werden können ⁸⁾.

Zusätzlich zu den erwähnten Peptiden oder Proteinen können immunmodulatorische Epitope beispielhaft ausgewählt aus Hepatitis B Virus oder anderen Viren, oder tumorassoziierte Antigene, in das Parapoxvirus einkloniert werden.

Damit wird eine starke, spezifische immunstimulatorische Eigenschaft gegen den Erreger oder den Tumor in das Parapoxvirus eingeführt.

Die Identifizierung geeigneter Epitope erfolgt mit bekannten,dem Fachmann geläufigen Techniken, wie beispielsweise der Flowzytometrie ⁹⁾.

Die Herstellung und Charakterisierung neuer rekombinanter Viren mit den beschriebenen Eigenschaften kann beispielhaft, wie im Folgenden aufgeführt, durchgeführt werden:

Herstellung eines rekombinanten Virus, dem Sequenzen fehlen, deren Genprodukte oder Teile davon nicht für die immunmodulatorische Wirkung öder für die Virusreplikation notwendig sind.

Ein Beispiel für die Klonierung des rekombinanten Parapoxvirus ovis geht von der Konstruktion von Doppelselektionskassetten aus, die ein Markergen, z. B. das LacZ-Gen unter Kontrolle des Vaccinia 11K-Gens oder einer anderen geeigneten Sequenz und ein anderes Selektionsmarkergen, z.B. das gpt-Gen (kodiert für das Enzym Xanthin-Guanin-Phosphoribosyl-Transferase, XGPRT) unter Kontrolle des entsprechend geeigneten Promotors exprimieren. Die Deletion viraler Sequenzen kann dann beispielhaft wie im Folgenden beschrieben, durchgeführt werden:

Singuläre Restriktionsschnittstellen in einem sowohl für die virale Replikation, als auch für die immunmodulatorische Wirkung nicht essentiellen Bereich von Parapoxvirus ovis, ein geeignetes Hüllprotein-Gen, ein anderes Gen, das für ein Strukturprotein kodiert (nachfolgend geeignetes Gen genannt) oder ein anderes Gen, z.B. VEGF-Gen, werden als Startpunkte verwendet, um eine bidirektionale Deletion von Sequenzen durch Einwirkung der Endonuklease Bal31 zu bewirken.

Hierzu wird beispielsweise das entsprechende Plasmid, ein geeignetes Strukturprotein-Gen, das die Nukleinsäuresequenz aus Parapoxvirus ovis enthält, im VEGF-Gen mit einem geeigneten Restriktionsenzym geöffnet und das nunmehr linearisierte Plasmid mit Bal31 inkubiert. Geeignete Deletionsplasmide werden aufgefüllt und hierauf komplementäre Oligonukleotide, die neue singuläre Schnittstellen, z.B. SmaI, SalI und EcoRV-Restriktionsschnittstellen darstellen, an die mit glatten Enden versehenen Bal 31-Produkte ligiert.

Nach der Transformation von Bakterien kann die Plasmid-DNA isoliert und mit einem Enzym gespalten werden, das in der Sequenz des entsprechenden Parapoxvirus ovis DNA-Fragmentes keine Erkennungsstelle enthält. Nach Insertion der mit den entsprechenden Restriktionsenzymen geschnittenen LacZ/gpt-Selektionskassette in die Deletionsstelle im geeigneten Gen kann die genaue Größe der erzeugten Deletionen in jeder resultierenden rekombinanten Plasmid-DNA durch Sequenzieren bestimmt werden.

Das Virus, dem dann das entsprechende Genprodukt oder eines Teiles davon fehlt, kann beispielhaft wie folgt hergestellt werden:
Konfluent ausgewachsene geeignete Zellen wie beispielsweise Rindernierenzellen werden mit einer Infektionsdosis von ca. 0,1 Multiplicity of infection (moi) infiziert. Nach etwa zwei Stunden werden die infizierten Zellen mit einem wie oben beschriebenen hergestellten Deletionsplasmid (z.B. 10 µg) beispielsweise unter Verwendung dem Fachmann geläufiger und kommerziell erhältlicher Transfektionssysteme transfiziert. Anschließend werden diese Zellkulturen mit einem geeigneten Selektionsmedium (z.B. mit HAT-Medium [Hypoxanthin-Aminopterin-Thymidin], MPA [Mycophenolsäure]) während 3 bis 6 Tagen bei ungefähr 37°C und etwa 5% CO2-Atmosphäre inkubiert bis ein zytopathischer Effekt (cpe) oder Plaquebildung sichtbar wird. Dann werden die Zellen lysiert, eine Verdünnungsreihe aus dem Zelllysat hergestellt und ein Plaque-Test auf geeigneten Zellen durchgeführt. Für den Plaquetest wird ein Agarosemedium-Gemisch zugegeben, das beispielsweise ungefähr 0,3 mg/ml Bluo-Gal (GIBCO) enthalten kann um blaue Plaques zu identifizieren, die z.B. LacZ-exprimierende, MPA-resistente rekombinante Viren enthalten. Die so erhaltenen rekombinanten Viren werden zur Infektion von geeigneten Zellen wie beispielsweise Rindernierenzellen, eingesetzt und mindestens zwei weiteren Plaquetitrationen unterzogen bis eine möglichst homogene, am günstigsten >99,9%ige, rekombinante Viruspopulation vorliegt.

Herstellung eines rekombinanten Virus, das Sequenzen enthält, deren Genprodukte oder Teile davon für ein organ-, gewebs- oder zellspezifisches Targeting notwendig sind.

Für die Herstellung des rekombinanten Virus mit Targetingsequenzen geht man analog vor. Als Ausgangsvirus wird ein wie oben beschrieben verändertes Virus verwendet. Alternativ kann der Einbau der Targetinsequenz in ein nicht genetisch verändertes Virus erfolgen, wenn die Virusreplikation und/oder die immun-modulatorische Wirkung nicht negativ beeinflusst werden. Anstelle des Plasmids, das deletierte oder verkürzte Sequenzen von Parapoxvirus ovis enthält, verwendet man ein entsprechendes Plasmid, das eine unveränderte oder in geeigneter Weise veränderte DNA-Sequenz enthält, die für ein Protein oder Peptid kodiert, das ein organ-, gewebs und/oder zellspezifisches Targeting des rekombinanten Virus in nicht inaktivierter oder in inaktivierter Form ermöglicht. Dies kann z.B. für den Fall, dass man das rekombinante Virus in die Leber bringen möchte, beispielsweise die Sequenz für das große Hüllprotein des Hepatitis B Virus des Menschen oder eine andere geeignete Sequenz sein. Erfolgt der Einbau der Targetingsequenz in ein Gen, das nicht für ein strukturelles Protein kodiert, so kann man die Targetingsequenz mit entsprechenden Membranankern koppeln, um einen Einbau in die Virushülle zu ermöglichen.

Die Wahl der Selektionsmarker ist bei der Herstellung so zu treffen, dass man nicht oder nur in geeigneter Art und Weise mit bereits vorhandenen Selektionsmarkern interferiert.

Analog können zusätzlich Sequenzen eingeführt werden, die für immunologisch aktive Epitope kodieren. Solche Epitope können mit dem Fachmann bekannten Methoden ausgewählt werden ⁹⁾.

Nachweis der Targetingeigenschaften des rekombinanten Virus.
Die neuen Eigenschaften des rekombinanten Virus werden einerseits bei diesem Virus mittels dem Fachmann bekannter geeigneter Methoden wie beispielsweise der Verwendung von Selektionsmarkern nachgewiesen und/oder dem Nachweis des neuen Proteins/Peptides erfolgt mittels Western-Blot. Andererseits kann ein funktioneller Nachweis erfolgen. Dieser wird an Zielzellen des Targeting geführt. Im Falle eines Lebertargeting mit einem rekombinanten Virus, das Asialoglycoprotein oder entsprechende Teile davon enthält kann dieser funktionelle Nachweis durch Bindung von rekombinantem Virus an Zellen, die den Asialoglycoprotein-Rezeptor exprimieren, nachgewiesen werden. Dies können humane Leberzellen oder Hepatomazellen (z.B. HepG2) sein, bei denen mit Asialoglycoprotein und rekombinantem Virus kompetitive Bindungsstudien durchgeführt werden können.

Zur Kontrolle erfolgen diese Studien auch an Zellen, die den Asialoglycoproteinrezeptor nicht exprimieren, beispielsweise Fibroblasten. Die Targetingeigenschaften sind sowohl bei inaktivierten als auch bei nicht inaktivierten rekombinanten Viren vorhanden. Für eine Therapie werden allerdings nur solche rekombinante Viren verwendet, bei denen die Targetingeigenschaften entsprechend der therapeutischen Zielstellung nachgewiesen werden konnten.

### Nachweis der immunmodulatorischen Eigenschaften

Der Nachweis der immunmodulatorischen Eigenschaften des rekombinanten Virus kann experimentell beispielsweise in Mäusen erfolgen. Dafür wird Mäusen beispielsweise Balb/c Mäusen, das rekombinante Virus in inaktivierter oder nicht inaktivierter Form beispielsweise in eine Körperhöhle, z.B. intraperitoneal oder subcutan, intramuskulär oder intravenös, injiziert. Nach einem festzulegenden Zeitschema, beispielsweise 6, 12, 24 Stunden nach der Applikation, werden die Tiere getötet, und es werden Organe und/oder Zellen, beispielsweise Zellen, die durch peritoneale Lavage gewonnen werden, entnommen. Aus den Organen/Zellen wird genetisches Material wie beispielsweise RNA isoliert und die Zytokinexpression mittels geeigneter Methoden wie beispielsweise durch Semiquantitative oder quantitative PCR bestimmt.

Für eine Therapie werden dann diejenigen rekombinante Viren verwendet, bei denen die immunmodulatorischen Eigenschaften (Induktion einer Th1 Immunantwort) einen therapeutischen Effekt erwarten lassen.

Unter Zugrundelegung der bekannten Zusammenhänge vom Einfluss einer Th1 Immunantwort auf latente und chronisch persistente Virusinfektionen ^{10,11)}
und der im Vergleich zu nichtrekombinantem Parapoxvirus ovis ähnlichen oder besseren immunmodulatorischen Eigenschaften des rekombinanten Parapoxvirus ovis ist der Einsatz von organ-, gewebs- und/oder zell-spezifischem rekombinanten Parapoxvirus ovis als Monotherapie oder in Kombination mit biologisch aktiven (z.B. antiviralen), niedermolekularen Verbindungen oder biologisch aktiven Proteinen an Mensch und Tier möglich und von therapeutischem Nutzen zur antiviralen Therapie von vorwiegend chronischen Infektionen mit dem Hepatitis B Virus; anderen viralen Infektionen der inneren Organe, namentlich der Leber, wobei beispielhaft das Hepatitis C Virus (HCV), oder alle anderen Erreger aus der Gruppe der Hepatitis verursachenden Viren genannt seien ¹²⁾, Infektionen, auch in Begleitung anderer Erkrankungen, mit den verschiedenen Typen des Herpes simplex Virus (HSV); den verschiedenen Typen von humanem Papillomvirus (HPV); dem humanem Immundefizienzvirus (HIV); dem humanem Cytomegaliecirus (HCMV); sowie den entsprechenden Viruserkrankungen beim Tier.

Des Weiteren können mit dem rekombinanten Parapox virus aufgrund des gezeigten Wirkmechanismus insbesondere die folgenden prophylaktischen oder therapeutischen Behandlungen erfolgversprechend durchgeführt werden:

Verhinderung von Rekurrenzen bei Herpesvirus-Infektionen, Metaphylaxe, d.h. Verhinderung der Etablierung von viralen Infektionen (z.B. HIV), wenn unmittelbar nach der Exposition mit dem Mittel behandelt wird ¹³⁾. Die Behandlung von Krebs ist aufgrund des Wirkmechanismus ebenfalls möglich ^{14, 15)}. Auch in diesen genannten Indikationen ist der Einsatz von organ-, gewebs- und/oder zellspezifischen rekombinanten Parapoxviren ovis als Monotherapie oder in sinnvoller Kombination mit biologisch aktiven, niedermolekularen Verbindungen möglich.

Die Behandlung entzündlicher und nichtentzündlicher degenerativer und proliferativer Erkrankungen der Leber wie beispielsweise der Leber-Zirrhose, und/oder der Leber-Fibrose mit rekombinantem Parapoxvirus ovis ist ebenfalls möglich. Auch in diesen genannten Indikationen ist der Einsatz von organ-, gewebs- und/oder zellspezifischen rekombinanten Parapoxviren ovis als Monotherapie oder in sinnvoller Kombination mit biologisch aktiven, niedermolekularen Verbindungen möglich.

Entsprechend der klinischen Fragestellung (z.B. chronische Hepatitis B Virus Erkrankung des Menschen) wird rekombinantes Virus zur organ- gewebs- und/oder zell-spezifischen Therapie hergestellt.

Man verfährt so, dass Gene, die nicht für eine Induktion einer zellvermittelten Immunantwort notwendig sind, deletiert oder mutiert werden. In diese Gene oder freien Genabschnitte setzt man dann die für Epitope (Peptide/Proteine) kodierenden Gensequenzen ein, die eine spezifische Interaktion mit einem oder mehreren Rezeptoren auf den Ziel-Zellen-Geweben oder -Organen gewährleisten. Alternativ kann man die für entsprechende Epitope kodierenden Gensequenzen in genetisch unveränderte Parapoxviren einsetzen, wenn die Virusreplikation, die Virusreifung und/oder die immunmodulaten Eigenschaften nicht negativ beeinflusst werden.

Zusätzlich kann durch geeignete immunologisch wirksame Epitope (z.B. Epitope des HBV), die zellvermittelte Immunantwort gegen einen Erreger spezifisch verstärkt werden.

Dazu wird das organ-, gewebs- und/oder zell-spezifisch interagierende/bindende rekombinante Parapoxvirus ovis zusätzlich mit spezifischen gegen einen oder mehrere Erreger gerichteten,- Immunantwort potenzierenden Epitopen ausgestattet und in der betreffenden Indikation eingesetzt (beispielsweise gegen eine oder mehrere der oben genannten Viruserkrankungen wie beispielsweise der chronische Hepatitis B Erkrankung des Menschen). Alternativ kann man die für entsprechende Epitope kodierenden Gensequenzen in genetisch unveränderte Parapoxviren einsetzen, wenn die Virusreplikation, die Virusreifung und/oder die immunmodulaten Eigenschaften nicht negativ beeinflusst werden:

Je nach klinischer Fragestellung bzw. ätiologisch beteiligtem Virus wird das rekombinante Parapoxvirus ovis in inaktivierter oder nicht inaktivierter Form systemisch (z.B. intramuskulär, subcutan, intraperitoneal, intravenös) oder lokal (z.B. in das betreffende Organ) appliziert.

Das rekornbinante Parapoxvirus ovis liegt dabei entweder lyophilisiert vor und wird unmittelbar vor der Applikation in einem geeignetem Lösungsmittel suspendiert oder aber es liegt in einer anderen geeigneten Formulierung vor.

Mehrere Applikationen bis hin zur kontinuierlichen Infusion nach Zeitschemen, die den Erfordernissen der klinischen Fragestellungen entsprechen, können dabei notwendig sein.

Der Einsatz von organ-, gewebs- und/oder zellspezifischen Parapoxviren ovis kann als Monotherapie oder in Kombination mit biologisch aktiven niedermolekularen Verbindungen entsprechend der Indikation und/oder der klinischen Fragestellung erfolgen.

Bei einer Kombination mit biologisch aktiven niedermolekularen Verbindungen kann die Applikation zeitgleich oder zeitlich versetzt erfolgen. So ist es beispielsweise möglich, zunächst mit einer niedermolekularen Verbindung (z.B. Nukleoidanalogen oder anderen Verbindungen) eine virale Replikation zu verringern oder zu verhindern und anschließend mit dem rekombinanten Parapoxvirus ovis eine virale Clearance herbeizuführen. Bei einer solchen Kombinationstherapie ist der Einsatz z.B. auch bei akuten viralen Infektionen möglich.

### Beispiel

### für die Herstellung und Testung einer Targeting-Mutante für den Herpes Virus Entry Mediator

Das Glycoprotein D (gD) des bovinen Herpesvirus 1 (BHV-1) ist an der Bindung des Virus an seine Zielzelle und für die Penetration des Virus in die Zielzelle verantwortlich, wobei hierbei noch andere virale Glycoproteine beteiligt sind (Liang et al. 1991). Neutralisierende gD spezifische Antikörper üben ihre Funktion aus, indem sie einen auf die Adsorption des Virus folgenden Schritt, die Penetration stören (Okazaki et al. 1986). gD dient somit als virale Bindungsstelle für den Herpes Virus Entry Mediator (HVEM) (Montgomery et al. 1996). Zellen, die diesen Herpesvirus Entry Mediator nicht besitzen, sind resistent gegen eine infektion mit verschiedenen Herpesviren, z.B. humanes Herpesvirus 1 [HSV-1] oder BHV-1. Verschiedene BHV-1-Stämme, die gD unterschiedlich stark exprimieren, penetrieren die Zellen unterschiedlich stark, wobei eine positive Korrelation zu dem gD Gehalt besteht (Fehler, 1991).

Rekombinantes Parapoxvirus ovis, das gD an der Oberfläche trägt, kann man nutzen, um solche HVEM-Bindungsstellen auf Zellen, die HVEM exprimieren (z.B. MDBK-Zellen) zu targeten. Infiziert man diese Zellen mit BHV-1, rekombinantem Parapoxvirus, welches gD exprimiert oder Wildtyp-Parapoxvirus, so sollte das Targeting des HVEM über die Penetrationsgeschwindigkeit meßbar sein. Dabei wird erwartet, dass gD rekombinantes Parapoxvirus etwa genausoschnell wie BHV-1, in jedem Falle jedoch schneller als Parapoxvirus ovis Wildtyp, der MDBK-Zellen ebenfalls infizieren kann, in die Zellen penetriert.

### Herstellung der LacZ Mutante:

In Parapoxvirus ovis (Strain D 1701) wurden die im Genom doppelt vorhandenen vegf-Gene nahezu vollständig deletiert und an diesen Stellen jeweils eine lacZ-xgpt Expressionskassette von E.coli eingefügt (Rziha et al. 1999).

### Herstellung des Transfektionsplasmides für die homologe Rekombination:

Das gD-Gen aus BHV1 einschließlich seiner Signalsequenz und Membrananker (Tikoo et al. 1990) wurde über PCR amplifiziert und blunt end in die EcoRV Site des Vektors pDVRec (Rziha et al. 1999) kloniert. Die Übereinstimmung der gD-Sequenz in pDVRec mit der Ursprungssequenz wurde durch Sequenzierung (MWG Biotech) bestätigt.

### Transfektion:

Die Transfektion der Parapoxvirus lacZ-Mutante (D1701-RV) erfolgte mit dem isolierten Plasmid pDVRec/gD und BKKL3A-Zellen. Bei einem Monolayer der Zellen von 70 bis 80 % (6-Well-Platte: Zellzahl von ca.4x10⁵ pro Well) wurde die Transfektion durchgeführt. Als Transfektionsreagenz diente das Liposomale Transfektionsreagenz DOSPER. Die Zellen wurden mit der Parapoxvirus lacZ-Mutante in einer MOI von 0,1 infiziert. 2µg Plasmid-DNA wurden im Verhältnis 1:3 und 1:4 mit DOSPER gemischt und nach der Virusinfektion den Zellen zugegeben.

4 bis 7 Tage nach der Transfektion zeigten die Zellen einen virus-spezifischen cytopathogenen Effekt und das Virus wurde durch dreimaliges Frieren und Tauen geerntet.

### Plaquereinigung:

BKKL3A Zellen wurden mit dem in Zehner Schritten verdünnten rekombinanten Virus (10⁻² bis 10⁻⁶) infiziert. Die Wells bei denen nach einigen Tagen ca.10 bis 30 beginnende Virusplaques zu sehen waren, wurden mit Agarose-Bluo-Gal 300 µg/ml (GIBCO) überschichtet. Nach 24 bis 48 h Inkubation bei 37°C (5 % CO2) erfolgte das Picken der weissen Plaques. Das Virusmaterial des ausgestanzten Agaroseblockes wurde über Nacht in Medium eluiert und das Virus erneut vermehrt (1.Plaquereinigung). Nach der ersten Plaquereinigung wurden die Klone im Dot Blot mit einer P³² markierten gD-DNA Sonde hybridisiert. Die Aufreinigung der positiven Rekombinanten erfolgt durch mindestens dreimalige Plaquereinigungsschritte.

### Penetrationsassay:

Bovine Nierenzellen (MDBK, ATCC No. CCL-22) wurden entsprechend den Anweisungen von ATCC gezüchtet und waren bei Versuchsbeginn konfluent. Die Zellen wurden 5 Minuten bei 4°C vorinkubiert. Anschließend wurde das Medium abgesaugt und vorgekühltes (4°C) a) BHV-1, b) gD rekombinantes Parapoxvirus oder c) Parapoxvirus ovis Wildtyp auf die Zellen gegeben (MOI 0.01). Danach wurden die Zellen 15 min bei 4°C inkubiert, das Medium abgesaugt und die Zellen 1x mit kaltem (4°C) PBS gewaschen. Im Anschluß daran wurden die Zellen weiter in 37°C warmen Medium weiter im Brutschrank inkubiert.

Nach 10, 20, 30, 60 und 120 Minuten wurde jeweils 1 well für ca. 45 Sekunden mit Zitratpuffer gewaschen, ein jeweiliges Kontrollwell mit PBS. Damit wurden die Viren, die bis dahin noch nicht in die Zellen penetriert sind, inaktiviert. Somit erhält man eine Penetrationskinetik. Die Zellen wurden nach den Säurebehandlungen mit Medium (das 0,5 % Methylzellulose enthält) überschichtet. Nach 3 Tagen wurden die Zellen fixiert, gefärbt und die Plaques mit einer Skala von 0 bis ++++ in einem Plaqueviewer bestimmt.

### Ergebnis:

Es zeigte sich, dass bei BHV-1 und gD rekombinantem Parapoxvirus ovis (gDPPVO) bereits nach ca. 7 Minuten mehr als die Hälfte der Viren die Cytoplasmamembran penetriert hat, während der Parapoxvirus ovis Wildtyp (wt PPVO) dafür ca. 20 Minuten benötigt. Diese Unterschiede sind signifikant (Varianzanalyse mit post hoc Vergleich). Damit konnte gezeigt werden, dass a) die Expression eines Proteins an der Oberfläche von Parapoxvirus ovis möglich ist und dass b) dieses Protein zum Targeten von spezifischen Rezeptoren, die wiederum auf bestimmten Zellen und/oder bestimmten Geweben exprimiert werden, benutzt werden kann.
1. Guidotti et al. (1996): Viral cross talk: Intracellular inactivation of the hepatitis B virus during an unrelated viral infection of the liver
2. Guidotti et al. (1994): Cytotoxic T lymphocytes inhibit hepatitis B virus gene expression by a noncytolytic mechanism in transgenic mice
3. Steinmassl,G., G.Wolf (1990): Bildung von Interleukin 2 und Interferon-γ durch mononukleäre Leukozyten des Schweines nach in vitro-Stimulation mit verschiedenen Viruspräparaten. J. Vet.Med.B37,5,321-331
4. Robinson, A.J. and Lyttle, D.J. (1992): Parapoxviruses: their biology and potential as recombinant vaccines. In: Recombinant Poxviruses, Chapter 9, 306-317 eds. M. Binns and G. Smith CRC Press, Boca Raton und WO 97/37031
5. Ishikawa, T. and Ganem, D. (1995): The pre-S domain of the large viral envelope protein determines host range in avian hepatitis B viruses. Proc. Natl.Acad. Sci. USA, 92 (14):6259-6263
6. Harris, J:D. and Lemoine, N.R. (1996): Strategies for targeted gene therapy. Trends in Genetics 12 (10): 400-405
7. Rensen, P.C.N., de Vrueh, L.A. and van Berkel, T.J.C.(1996): Targeting Hepatitis B Therapy to the Liver. Clin. Pharmacokinet. 31 (2)131-155
8. Barry, B.A., Dower, W.J. and Johnston, S.A. (1996): Toward cell-targeting gene therapy vectors: Selection of cell-binding peptides from random peptide-presenting phage libraries. Nature Medicine 2 (3):299-305
9. Kern, F., Surel, I.P., Brock, C., Freistedt, B., Radtke, H., Scheffold, A., Blasczyk, R., Reinke, P., Schneider-Mergener, J., Radbruch, A., Walden, P., Volk, H.D. (1998): T-cell epitope mapping by flow cytometry. Nat. Med. 4 (8): 975-978
10. P. Lucin, S. Jonjic, M. Messerle, B. Polic, H. Hengel, U.H. Koszinowski (1994): Late-Phase Inhibition of murine cytomegalovirus replication by synergistic action of interferon gamma and tumor necrosis factor alpha. J. Gen. Virol 75:101-110; P.M.
11. Smith, R.M. Wolcott, R. Chervenak, S.R. Jennings (1994): Control of acute cutaneous herpes-simplex virus-Infection - T-cell mediated viral clearance is dependent upon interferon gamma. Virology 202 (1):76-88]
12. Y. Kawanashi, N. Hayashi, K. Katayama, K. ueda, T. Takehara, E. Miyoshi, E. Mita, A. Kasahara, H. Fusamoto, T. Kamada (1995): Tumor necrosis factor alpha and interferon gamma inhibit synergistically viral replication in hepatitis B virus replicating cells. J. Medical Virology 47 (3):272-277
13. Dhawan, S., L.M. Wahl, A. Heredia, Y.H. Zhang, J.S. Epstein, M.S. Meltzer, I.K. Hewlett (1995): Interferon gamma inhibits HIV-induced invasiveness of Monocytes. J. Leukocyte Biology, 58 (6):713-716
14. J.F. Bromberg, C.M. Horvath, Z.L. Wen, R.D. Schreiber, J.E.Damell (1996): Transcriptionally active statl is required for the antiproliferative effects of both interferon alpha and interferon gamma. PNAS 93(15):7673-7678;
15. M.Klouche, H.Kirchner, F.Holzel (1994): Antiproliferative effects of interferon gamma in combination with alpha-difluoromethylornithine on human carcinoma cell cultures. J.Cancer Research and Clinical Oncology 1120(12):706]
16. Fehler F (1991): Glycoprotein IV des bovinen Herpesvirus 1: funktionelle und strukturelle Eigenschaften eines essentiellen herpesviralen Glycoproteins Dissertation, Eberhard-Karls-Universität Tübingen
17. Liang X, Babiuk L A, van Drunen, Little, Van den Hurk S, Fitzpatrick D R, Zamö T J (1991): Bovine herpesvirus 1 attachment to permissive cells is mediated by ist major glycoproteins gI, gIII and gIV. J. Virol. 65:1124-1132.
18. Montgomery R I, Warner M W, Lum B J, Spear P G (1996) Cell 87:427
19. Okazaki K, Honda E, Minetoma T, Kumagai T (1986): Mechanisms of neutralization by monoclonal antibodies to different antigenic sites on the bovine herpesvirus type 1 glycoproteins. Virology 150:260-264
20. Rziha H-J, Henkel M, Cottone R, Meyer M, Dehio C, Büttner M (1999): Parapoxviruses:potential alternative vectors for directing the immune response in permissive and non-permissive hosts. Journal of Biotechnology, Vol. 73,235-242
21. Rziha H-J, Henkel M, Cottone R, Bauer B, Auge U, götz F, Pfaff E, Büttner M (1999): Generation of recombinant Parapoxviruses: Non-essential genes suitable for foreign gene expression. Journal of Biotechnology (1.09.99) ??
22. Tikoo S K, Fitzpatrick D R, Babiuk 1 A, Zamb T J (1990): Molecular Cloning, Sequencing, and Expression of functional bovine Herpesvirus 1 Glycoprotein gIV in Transfected Bovine Cells . Journal of Virology, Vol. 64, No.10, 5132-5142

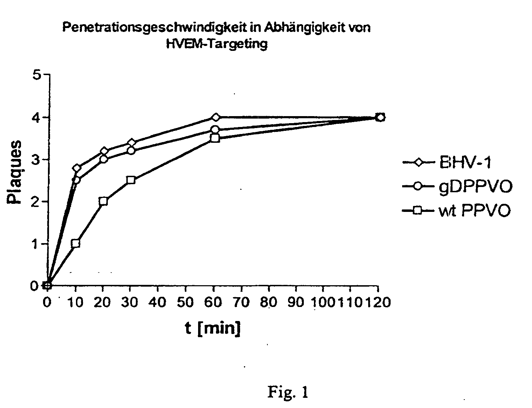

## Patentansprüche

1. Verwendung von rekombinantem Parapoxvirus mit Targetingeigenschaften zur Herstellung von Arzneimitteln.

2. Arzneimittel enthaltend rekombinantes Parapoxvirus mit Targetingeigenschaften.

## Claims

1. Use of recombinant parapoxvirus possessing targeting properties for producing pharmaceuticals.

2. Pharmaceutical which comprises recombinant parapoxvirus possessing targeting properties.

## Revendications

1. Utilisation de parapoxvirus recombiné possédant des propriétés de targeting pour la préparation de médicaments.

2. Médicament contenant un parapoxvirus recombiné possédant des propriétés de targeting.
